# EUROPEAN PATENT APPLICATION

(11) **EP 3 756 727 A1**
(43) Date of publication of application: **30.12.2020**
(21) Application number: 19182283.2
(22) Date of filing: 25.06.2019
(51) Int. Cl.: A61N 2/02, A61N 2/00, A61B 5/055, G01R 33/34, A61B 5/11, G01R 33/28, G01R 33/385

(54) **STIMULATION APPARATUS**

(71) Applicant: Koninklijke Philips N.V., 5656 AG Eindhoven (NL)
(72) Inventor: WEISS, Steffen, 5656 AE Eindhoven (NL); JOHNSON, Mark Thomas, 5656 AE Eindhoven (NL); HELLE, Michael Günter, 5656 AE Eindhoven (NL)
(74) Representative: de Haan, Poul Erik

(57) **Abstract**

The present invention relates to a stimulation apparatus (10), comprising a magnetic stimulation unit (20), and a processing unit (30). The processing unit is configured to control the magnetic stimulation unit to provide intentional nerve and/or muscle stimuli to a peripheral body part of a patient.

## Description

### FIELD OF THE INVENTION

The present invention relates to a stimulation apparatus, an image acquisition system, a magnetic resonance imaging system, a method of stimulating a patient, and a method of image acquisition with a magnetic resonance imaging system, as well as to a computer program element and a computer readable medium.

### BACKGROUND OF THE INVENTION

Interaction with patients in medical imaging environments such a where a Magnetic Resonance image (MRI) acquisition unit is being utilized, or where a Computer Tomography (CT) image acquisition unit is being utilized, or where a Positron Emission Tomography (PET) image acquisition unit is being utilized, or where a digital X-ray Radiogrammetry (DXR) image acquisition unit is being utilized, is challenging. For example, the identification of the objective sedation status is one of the key problems for the imaging to select the right protocol with an adapted timing sequence and also to identify how the sedation status is changing to decide on next steps. It is particularly challenging to assess the sedation state in the noisy environment of a scanner system. Other situations where interaction with the patient is desired are for example repositioning a patient, calming an anxious patient, helping a patient to hold their breath, all of which are difficult in the noisy and busy environments of such scanner systems.

There is a need to address these issues.

### SUMMARY OF THE INVENTION

It would be advantageous to have improved means of interacting with a patient undergoing a medical scan. The object of the present invention is solved with the subject matter of the independent claims, wherein further embodiments are incorporated in the dependent claims. It should be noted that the following described aspects and examples of the invention apply also to the stimulation apparatus, the image acquisition system, the magnetic resonance imaging system, the method of stimulating a patient, and the method of image acquisition with a magnetic resonance imaging system, as well as to the computer program element and a computer readable medium.

In a first aspect, there is provided a stimulation apparatus, comprising:
- a magnetic stimulation unit; and
- a processing unit.

The processing unit is configured to control the magnetic stimulation unit to provide intentional nerve and/or muscle stimuli to a peripheral body part of a patient.

In an example, the apparatus comprises at least one magnetic stimulation coil. The processing unit is configured to control the at least one magnetic stimulation coil to provide the intentional nerve and/or muscle stimuli to the patient in a predefined spatial and/or temporal manner.

In an example, the processing unit is configured to select at least one part of one magnetic stimulation coil of the at least one magnetic stimulation coil to provide the intentional nerve and/or muscle stimuli to the patient in the predefined spatial manner.

In an example, the at least one magnetic stimulation coil comprises a plurality of magnetic stimulation coils. The processing unit is configured to select at least a part of one or more magnetic stimulation coils of the plurality of magnetic stimulation coils to provide the intentional nerve and/or muscle stimuli to the patient in the predefined spatial manner.

In an example, the processing unit is configured to control the magnetic stimulation unit to provide intentional nerve and/or muscle stimuli to a plurality of different locations of the patient.

In an example, the processing unit is configured to control a waveform of a current applied to the at least one magnetic stimulation coil to provide the intentional nerve and/or muscle stimuli to the patient in the predefined temporal manner.

In an example, the apparatus comprises a plurality of magnetic stimulation coil drive amplifiers, and the at least one magnetic stimulation coil comprises a plurality of magnetic stimulation coils. Each magnetic stimulation coil is configured to be driven by at least one amplifier, wherein each amplifier is configured to drive only one magnetic stimulation coil, and wherein the processing unit is configured control the plurality of amplifiers to provide the intentional nerve and/or muscle stimuli to the patient in the predefined spatial and/or temporal manner.

In an example, a first magnetic stimulation coil is configured to be driven by a first amplifier and a second magnetic stimulation coil is configured to be driven by a second amplifier. In an example, a third magnetic stimulation coil is configured to be driven by a third amplifier.

In this way, one coil can be driven by an amplifier to provide a single gradient (e.g. an x-gradient). However, when one coil is driven by one amplifier and a second coil is driven by a second amplifier two independent gradients can be generated (e.g. x,y). And, when a third coil is driven by a third amplifier a third independent gradient can be generated (x,y,z). It is to be noted that the amplifiers can provide independent magnetic stimulation fields (x,y,z) that need not be gradients as such.

In an example, the processing unit is configured to control the magnetic stimulation unit to provide intentional nerve and/or muscle stimuli to the patient to provide information to the patient.

In an example, the apparatus is configured to acquire at least one patient response to the nerve and/or muscle stimuli. The processing unit is configured to determine a sedation state of the sedated patient comprising utilization of the at least one patient response to the nerve and/or muscle stimuli.

In a second aspect, there is provided an image acquisition system, comprising:
- an image acquisition unit; and
- a stimulation apparatus according to the first aspect.

The image acquisition unit is configured to acquire image data of a patient. The stimulation apparatus is configured to provide intentional nerve and/or muscle stimuli to a peripheral body part of the patient

In a third aspect, there is provided a magnetic resonance imaging system, comprising:
- a stimulation apparatus according to the first aspect.

The magnetic resonance image acquisition system is configured to acquire image data of the patient. The processing unit of the stimulation apparatus is configured to interleave a waveform used for the intentional nerve and/or muscle stimuli of the stimulation apparatus with a waveform used for Magnetic Resonance imaging of the magnetic resonance imaging system.

In a fourth aspect, there is provided a method of stimulating a patient, comprising:
controlling by a processing unit a magnetic stimulation unit to provide intentional nerve and/or muscle stimuli to a peripheral body part of a patient.

In a fifth aspect, there is provided a method of image acquisition with a magnetic resonance imaging system, the method comprising:
- providing intentional nerve and/or muscle stimuli to a patient by a magnetic stimulation unit according to the method of the fourth aspect;
- acquiring image data of the patient with the magnetic resonance imaging system; and
- interleaving by the processing unit a waveform used for the intentional nerve and/or muscle stimuli with a waveform used for Magnetic Resonance imaging.

According to another aspect, there is provided a computer program element controlling one or more of the apparatuses as previously described which, if the computer program element is executed by a processing unit, is adapted to perform one or more of the methods as previously described.

According to another aspect, there is provided a computer readable medium having stored computer element as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a stimulation apparatus;
Fig. 2 shows a schematic set up of an example of an image acquisition;
Fig. 3 shows a schematic set up of an example of a magnetic resonance imaging system;
Fig. 4 shows an example of a method of stimulating a patient;
Fig. 5 shows an example of a method of image acquisition with a magnetic resonance imaging system;
Fig. 6 shows a schematic representation of gradient coils for an MR image acquisition unit or scanner; and
Fig. 7 shows a representation of an example of a y-gradient coil.

### DETAILED DESCRIPTION OF EMBODIMENTS

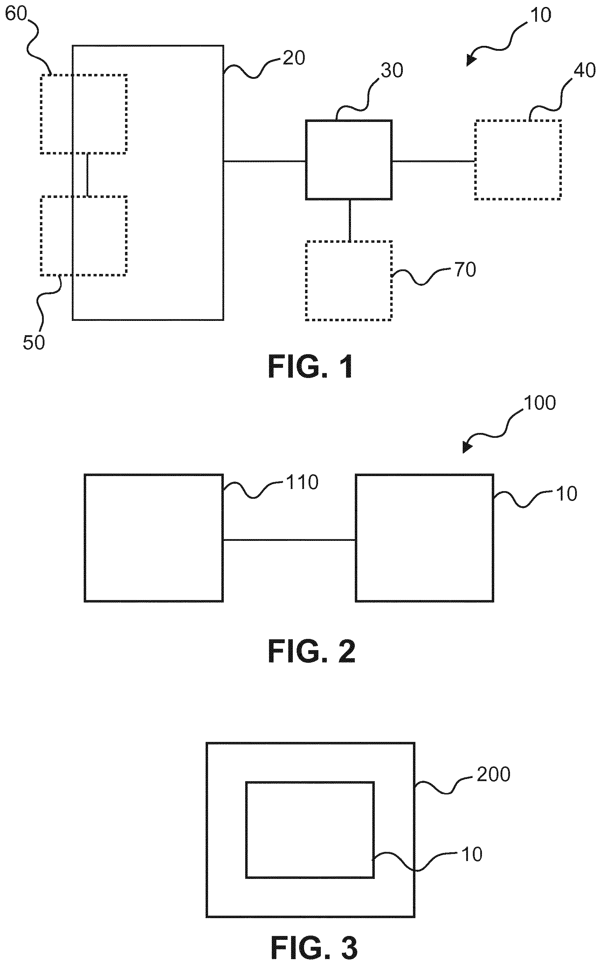

Fig. 1 shows an example of a stimulation apparatus 10, comprising a magnetic stimulation unit 20, and a processing unit 30. The processing unit is configured to control the magnetic stimulation unit to provide intentional nerve and/or muscle stimuli to a peripheral body part of a patient.

In an example, the magnetic stimulation unit is at least part of a magnetic resonance image acquisition unit

In an example, the stimulation apparatus comprises at least one sensor device 40 configured to acquire at least one response to the nerve and/or muscle stimuli.

In an example, the at least one sensor device comprises: a camera, an EMG sensor, a movement sensor, a tilt sensor, an accelerometer, a microphone, and the at least one sensor device can be the magnetic resonance image acquisition unit itself when operating in an image acquisition mode.

In an example, the peripheral body part comprises a part of the leg, a part of the foot, a part of the arm, a part of the hand.

In an example, the peripheral body part means any part of the body other than the head, and includes for example the back/spine.

In an example, the processing unit is configured to implement a specific instruction set to provide the intentional nerve and/or muscle stimuli to the patient.

According to an example, the apparatus comprises at least one magnetic stimulation coil 50. The processing unit is configured to control the at least one magnetic stimulation coil to provide the intentional nerve and/or muscle stimuli to the patient in a predefined spatial and/or temporal manner.

In an example, the at least one magnetic stimulation coil is part of the magnetic resonance image acquisition unit.

In an example, the at least one magnetic stimulation coil comprises at least one gradient coil.

According to an example, the processing unit is configured to select at least one part of one magnetic stimulation coil of the at least one magnetic stimulation coil to provide the intentional nerve and/or muscle stimuli to the patient in the predefined spatial manner.

According to an example, the at least one magnetic stimulation coil comprises a plurality of magnetic stimulation coils. The processing unit is configured to select at least a part of one or more magnetic stimulation coils of the plurality of magnetic stimulation coils to provide the intentional nerve and/or muscle stimuli to the patient in the predefined spatial manner.

In an example, the at least one magnetic stimulation coil is represented by at least one gradient coil of an MRI unit or system.

In an example, reference to a magnetic stimulation coil can refer to a part of a gradient coil of an MRI unit or system.

According to an example, the processing unit is configured to control the magnetic stimulation unit to provide intentional nerve and/or muscle stimuli to a plurality of different locations of the patient.

According to an example, the processing unit is configured to control a waveform of a current applied to the at least one magnetic stimulation coil to provide the intentional nerve and/or muscle stimuli to the patient in the predefined temporal manner.

In an example, the applied current has a high maximum current amplitude.

In an example, the waveform comprises a bipolar trapezoidal gradient waveform.

In an example, the processing unit is configured to provide pulses of nerve and/or muscle stimuli, wherein the pulses have durations of the order of 0.1ms to 100ms.

According to an example, the apparatus comprises a plurality of magnetic stimulation coil drive amplifiers 60. The at least one magnetic stimulation coil comprises a plurality of magnetic stimulation coils. Each magnetic stimulation coil is configured to be driven by at least one amplifier. Each amplifier is configured to drive only one magnetic stimulation coil. The processing unit is configured to control the plurality of amplifiers to provide the intentional nerve and/or muscle stimuli to the patient in the predefined spatial and/or temporal manner.

According to an example, a first magnetic stimulation coil is configured to be driven by a first amplifier and a second magnetic stimulation coil is configured to be driven by a second amplifier.

In an example, a third magnetic stimulation coil is configured to be driven by a third amplifier.

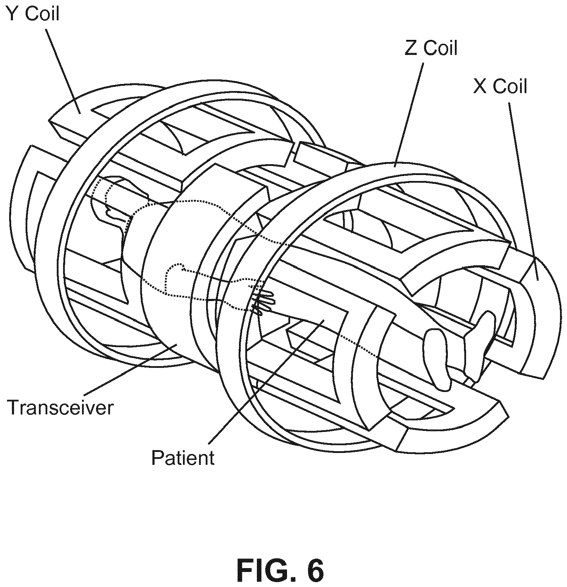

In the above discussion, a coil can mean a single coil. However, a coil can also mean a coil device having several individual coils. Thus, part of a coil can refer to coil of a coil device that has a number of coils. This is explained further with reference to Fig. 6 below.

According to an example, the processing unit is configured to control the magnetic stimulation unit to provide intentional nerve and/or muscle stimuli to the patient to provide information to the patient.

In an example, the information provided to the patient enables the patient to reposition at least one part of the patient within the magnetic resonance image acquisition unit.

In an example, the information provided to the patient relates to breathing guidance.

In an example, the information provided to the patient is configured to calm the patient. In an example, the information provided to the patient to calm the patient comprises nerve and/or muscle stimuli suggestive of a reassuring caress or touch from a caregiver.

According to an example, the apparatus is configured to acquire at least one patient response to the nerve and/or muscle stimuli. The processing unit is configured to determine a sedation state of the sedated patient comprising utilization of the at least one patient response to the nerve and/or muscle stimuli.

In an example, the apparatus comprises an output unit 70 configured to output the sedation state of the sedated patient.

In other words, a sedation level determination system is provided that can determine the sedation level of a patient for utilization in for example a medical scan procedure.

In an example, the output unit can be used to adapt a medical scan procedure based on the responses.

Fig. 2 shows an example of an image acquisition system 100, comprising an image acquisition unit 110, and a stimulation apparatus 10 as described with respect to Fig. 1. The image acquisition unit is configured to acquire image data of a patient. The stimulation apparatus is configured to provide intentional nerve and/or muscle stimuli to a peripheral body part of the patient.

In an example, the image acquisition unit is: a Magnetic Resonance image acquisition unit (MRI); a Computer Tomography image acquisition unit (CT), a Positron Emission Tomography image acquisition unit (PET), a digital X-ray Radiogrammetry image acquisition unit (DXR), or any other medical image acquisition unit.

In an example, the processing unit is configured to determine at least one scan protocol and/or terminate at least one scan protocol for the image acquisition unit for the acquisition of the image data comprising utilization of a determined sedation state of the patient.

Thus a new and effective way of interacting with patients, in a challenging medical imaging environment, is provided.

Fig. 3 shows an example of a magnetic resonance imaging system 200, comprising a stimulation apparatus 10 as described with respect to Fig. 1. The magnetic resonance image acquisition system is configured to acquire image data of the patient. The processing unit of the stimulation apparatus is configured to interleave a waveform used for the intentional nerve and/or muscle stimuli of the stimulation apparatus with a waveform used for Magnetic Resonance imaging of the magnetic resonance imaging system.

Thus, it is enabled to interact with a patient in the bore of an MRI scanner in a tactile manner without the requirement of additional devices for this. Therefore, peripheral nerve stimulation is applied in a controlled way using the gradient coil system of the MR system itself.

In an example, the stimulation apparatus is comprised within the magnetic resonance image acquisition unit.

In an example, the coils of stimulation apparatus are the coils of the magnetic resonance image acquisition system that are used as part of MR imaging.

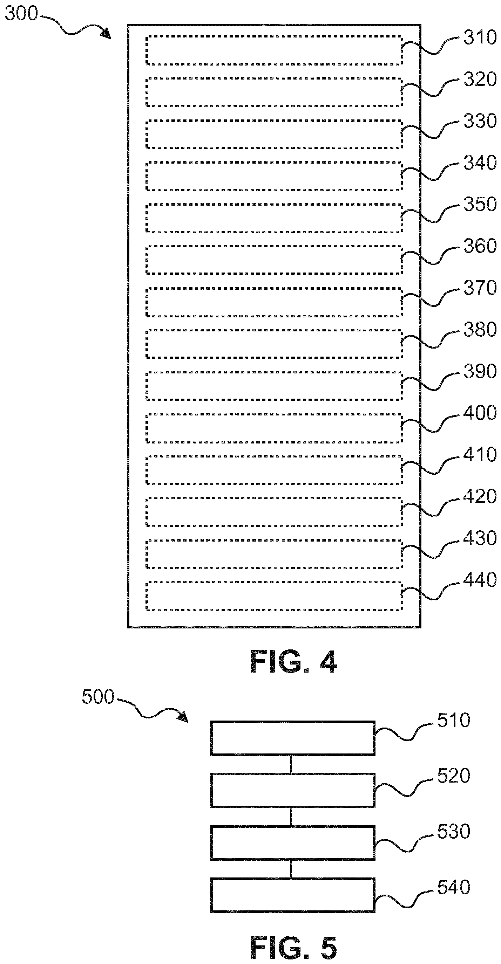

Fig. 4 shows a method 300 of stimulating a patient, comprising:
controlling 310 by a processing unit a magnetic stimulation unit to provide intentional nerve and/or muscle stimuli to a peripheral body part of a patient.

In an example, the magnetic stimulation unit is at least part of a magnetic resonance image acquisition unit

In an example, the method comprises acquiring by at least one sensor device at least one response to the nerve and/or muscle stimuli.

In an example, the method comprises implementing 320 by the processing unit a specific instruction set to provide the intentional nerve and/or muscle stimuli to the patient.

In an example, the method comprises controlling 330 by the processing unit at least one magnetic stimulation coil to provide the intentional nerve and/or muscle stimuli to the patient in a predefined spatial and/or temporal manner.

In an example, the at least one magnetic stimulation coil is part of the magnetic resonance image acquisition unit.

In an example, the at least one magnetic stimulation coil comprises at least one gradient coil.

In an example, the method comprises selecting 340 by the processing unit at least one part of one magnetic stimulation coil of the at least one magnetic stimulation coil to provide the intentional nerve and/or muscle stimuli to the patient in the predefined spatial manner.

In an example, the method comprises selecting 350 at least one part of one or more magnetic stimulation coils of a plurality of magnetic stimulation coils to provide the intentional nerve and/or muscle stimuli to the patient in the predefined spatial manner.

In an example, the method comprises controlling 360 the magnetic stimulation unit to provide intentional nerve and/or muscle stimuli to a plurality of different locations of the patient.

In an example, the method comprises controlling 370 a waveform of a current applied to the at least one magnetic stimulation coil to provide the intentional nerve and/or muscle stimuli to the patient in the predefined temporal manner.

In an example, the applied current has a high maximum current amplitude.

In an example, the waveform comprises a bipolar trapezoidal gradient waveform.

In an example, the method comprises providing 380 pulses of nerve and/or muscle stimuli, wherein the pulses have durations of the order of 0.1ms to 100ms.

In an example, each magnetic stimulation coil of a plurality of magnetic stimulation coils is configured to be driven by at least one amplifier of a plurality of magnetic stimulation coil drive amplifiers. Each amplifier is configured to drive only one magnetic stimulation coil. The method can then comprise controlling 390 by processing unit the plurality of amplifiers to provide the intentional nerve and/or muscle stimuli to the patient in the predefined spatial and/or temporal manner.

In an example, the method comprises driving each magnetic stimulation coil by at least two different amplifiers.

In an example, the method comprises controlling 400 the magnetic stimulation unit to provide intentional nerve and/or muscle stimuli to the patient to provide information to the patient.

In an example, the information provided to the patient enables the patient to reposition at least one part of the patient within the magnetic resonance image acquisition unit.

In an example, the information provided to the patient relates to breathing guidance.

In an example, the information provided to the patient is configured to calm the patient. In an example, the information provided to the patient to calm the patient comprises nerve and/or muscle stimuli suggestive of a reassuring caress or touch from a caregiver.

In an example, the method comprises acquiring 410 at least one patient response to the nerve and/or muscle stimuli; and determining 420 by the processing unit a sedation state of the sedated patient comprising utilization of the at least one patient response to the nerve and/or muscle stimuli.

In an example, the method comprises outputting by an output unit the sedation state of the sedated patient.

In an example, the method comprises adapting a medical scan procedure based on the at least one response.

In an example, method comprises determining 430 by the processing unit at least one scan protocol and/or terminating 440 at least one scan protocol for an image acquisition unit for the acquisition of the image data comprising utilization of a determined sedation state of the patient.

Fig. 5 shows a method 500 of image acquisition with a magnetic resonance imaging system, the method comprising:
- providing 510 intentional nerve and/or muscle stimuli to a patient by a magnetic stimulation unit according to the method described with respect to Fig. 4;
- acquiring 520 image data of the patient with the magnetic resonance imaging system; and
- interleaving 530 by the processing unit a waveform used for the intentional nerve and/or muscle stimuli with a waveform used for Magnetic Resonance imaging.

In an example, the magnetic stimulation unit is comprised within the magnetic resonance image acquisition unit.

The stimulation apparatus, the image acquisition system, the magnetic resonance imaging system, the method of stimulating a patient, and the method of image acquisition with a magnetic resonance imaging system are now described in more detail with respect to specific detailed embodiments.

It was realised by the inventors that the effect known as peripheral nerve stimulation (PNS) could be used beneficially in a medical imaging environment. The strong currents applied to the Magnetic resonance gradient coils during a MRI procedure are known to have an undesirable side effect, which excites sensorial and motor nerves in the patient. The patient feels this as a tickling sensation or spontaneous slight muscle contraction typically at the arms or the back. As discussed this effect is normally considered to be undesirable and is avoided if at all possible during an MRI scan. Standard MRI systems have three independent gradient coils X,Y,Z, and each coil consists of several coil parts connected in series so that all parts carry the same current are driven by one of the gradient coil amplifiers X,Y,Z. The inventors realised that the PNS effect could be intentionally utilized to stimulate a patient during an MRI scan, where parts of an MRI image acquisition unit could be utilized and with modifications further beneficial effects could be provided. It was also realised that a dedicated magnetic stimulation apparatus, usable to stimulate the patient, could be used as an add-on to normal scanning with CT, PET and DXR for example.

The following discussion centres on an MRI imaging environment, however as made clear above, the apparatus, systems and methods have wider utility in other imaging environments such as CT, PET and DXR. Thus, a tactile interaction with the patient, where a tactile sensation in the patient is created, is provided that would otherwise not be practical without additional devices. This is especially the case in closed bore systems as MRI systems, as there is no direct access to the bore during the scanning procedure. This is also generally the case in an autonomous imaging environment, where a minimum of staff or no staff at all is present for direct human interaction with the patient.

In an MRI environment, the inventors realized that it is possible to make use of the undesirable PNS side effect of MR imaging as a basis of introducing a tactile communication path to the patient in the scanner. Thus, instead of trying to reduce the amount of PNS felt by the patient, the inventors have introduced a new technique, that could involve new MR scan sequences, that intentionally induces PNS in the patient in a controlled manner. Thus, in an MRI system, at least parts the gradient coils used for MRI can be used for intentional magnetic stimulation. Therefore, strong currents are applied to the MR gradient coils to induce PNS such that the waveforms of these currents are interleaved with the waveforms of the scan sequence used for MRI. Details of the conditions for interleaving are known to persons skilled in MR sequence design and in part described in the embodiments below. The strong currents are used to excite sensorial and motor nerves in the patient in a predefined spatial and temporal manner. Temporal behavior is governed by the waveforms of the injected currents. Spatial behavior is governed by the selection of particular coils for example with three coils with three associated amplifiers being used to generate a triple gradient (x, y, and z). It is to be noted that even a particular part of the gradient coils can be used, and where a single coil and amplifier can generate a single gradient e.g. x, y or z. Thus, two coils with two amplifiers can be used to generate a double gradient e.g. (x, y), or (x, z), or (y, z). Consequently, the patient feels this as a localized tactile sensation, for example a tickling sensation or spontaneous slight muscle contraction localized for example at the arms or the back. By inducing either single or multiple tactile signals at one or more location a series of interactions can be realized. This is discussed in more detail in the specific embodiments below.

The following detailed embodiments provide further details on how the stimulation apparatus, the image acquisition system, the magnetic resonance imaging system, the method of stimulating a patient, and the method of image acquisition with a magnetic resonance imaging system can be realised as would be appreciated by the skilled person.

### Embodiment 1: Suitable waveforms for inducing tactile interaction via PNS and their integration with the MR scan

Typically bipolar trapezoidal gradient waveforms with high current amplitude and pulse durations in the order of 0.1ms to 100ms are applied to the gradient coils. These can be interleaved with waveforms used for MR imaging using known techniques of the art without compromising the MR imaging. Interleaving here is meant in a wide sense, defined as follows: a stimulation pulse can be played out at all times during MR scanning on any gradient coil or any part of a gradient coil except during times of RF pulse transmission and times of MR signal reception. As known in MR sequence design, such close interleaving with the MR scan also requires that the waveform of the stimulation pulse fulfills the condition that its current integral over time equals zero at the end of the stimulation pulse, e.g., by using a bipolar stimulation pulse with equal negative and positive lobes. The amplitude of the PNS pulses can be varied over time such that the strength of the sensation by the patient varies. In some embodiments the strength of the sensation could be made to vary in synchronicity with the concurrent multi-sensorial stimuli. It is to be noted that PNS can be induced in all patients using the described techniques, but the sensitivity to PNS varies from patient to patient. Therefore, it has been found to be beneficial to first assess a patient for his/her sensitivity to PNS and also for their values (tolerance, sensitivity etc.) and create a personalized sensory response model.

### Embodiment 2: Suitable selection of coil and coil part for induction of PNS

Fig. 6 shows a schematic representation of the gradient coils for an MR imaging acquisition unit or scanner. Every gradient coil and coil part produces a characteristic magnetic field distribution that results from the spatial arrangement of the coil leads. Therefore, every gradient coil (X,Y,or Z) also has a particular distribution of locations where PNS occurs. This is used to deliver tactile sensations at different locations. Additionally, in a specific embodiment coil parts of one coil device are connected to separate amplifiers. Here, a coil device for example can be formed from two separate coils, three separate coils or four separate coils. In standard MR systems, all coil parts of one gradient coil device are connected in series and this series is connected to one amplifier only. Thus, for example in a standard MRI the current in the two separate coil parts of the z coil device flows in an anti-parallel direction and can be powered by one amplifier. Only rarely, each coil part is driven by a separate amplifier. If so, this is done only to drive the gradient coil faster for faster MR imaging and all amplifiers produce the same or almost the same waveform. In the present technique however, coil parts are connected to separate amplifiers to drive currents with very different waveforms through these parts. Thus, in the present technique the two z coils, as shown in Fig. 6, can be driven by separate amplifiers, and indeed only one of the two coils can be activated to induce PNS intentionally in the patient. Therefore, in a simple example, a strong current can be driven only through part one of two coil parts of a coil device, whereas coil part two of that coil device does not carry any current. This is used to induce PNS only in the body part that is subjected to the magnetic field of coil part one. Effectively, this can be used to selectively induce PNS at certain locations in the body in a more focused way than with using entire gradient coils only. This also means that in the present technique the x coil device, that has four separate coils, can also have four separate amplifiers to drive each coil part individually, and where one, two, three or all four coils can be activated to intentionally induce PNS. However, the normal coils of an MRI scanner can be utilized to intentionally induce PNS in a patient.

Interleaving of stimulation pulses with MR scanning according to the timing and waveform conditions described in embodiment 1 even may include the following two cases or variants thereof: in a first case, while all four parts of the x-gradient coil play out a particular waveform required for MR scanning, a stimulation pulse may be played out on at least one part of the y-gradient coil or the z-gradient coil. In a second case, while all four parts of the x-gradient coil play out a particular waveform required for MR scanning, a stimulation pulse may be played out on at least one part of the x-gradient coil itself.

Thus, referring to Fig. 6, the coils as described above, can be made smaller and if necessary be localized for only a part of a patient, and form a stimulation apparatus that can operate in conjunction with an image scanner such as CT, PET, attenuation X-ray etc., to intentionally induce PNS in patients.

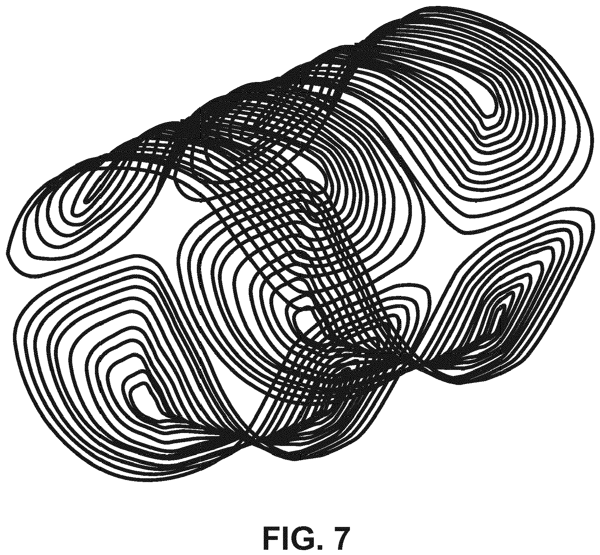

It is to be noted that the representation of coils in Fig. 6 is schematic only, and the coils are actually very intricately shaped as shown in Fig. 7, which shows a y-gradient coil.

### Embodiment 3: Nerve and muscle stimulation for determining state of sedation

In the third embodiment tactile sensation induced by PNS is used to act as a stimulus to assess the sedation level of a patient without having to physically touch the patient. In this embodiment any of the known sedation level assessment methods maybe used to assess the response of the patient to the PNS stimulus and hence assess the sedation state. This can involve for example a manual assessment, or a sensor based assessment, with automated sedation state determination. Here, the PNS can be applied a single or multiple time to the same or different parts of the body. Also the intensity of the PNS (due to the current amplitude in the coils) can be scanned to assess a level of sedation and potentially to track the sedation level during the course of the MRI examination. Feedback to dosage of sedation medication can also be provided.

### Adaptation of the scan sequence

Given that each sedation state has a corresponding duration, that may be measured based on patient's response, a specific scan sequence is automatically adjusted so that a right level of sequence can be prioritized considering the patient conditions.

### Embodiment 4: Nerve and muscle stimulation for repositioning patient

In the fourth embodiment the tactile sensation induced by PNS is used to act as a stimulus to reposition a patient in the bore of the MRI scanner without having to physically touch the patient. In this embodiment a camera, direct vision or any known method can be used to assess the response of the patient to the PNS stimulus and see if repositioning was successful. If further repositioning is required, the PNS can be again be applied singly or multiple times to the same or different parts of the body. Also the intensity of the PNS (the current amplitude in the coils) can be altered to suggest to the patient that for example smaller or larger movements are required.

### Embodiment 5: Nerve and muscle stimulation for breathing guidance

In the fifth embodiment the tactile sensation induced by PNS is used to act as a stimulus to guide the breathing of a patient in the bore of the MRI scanner without having to physically touch the patient. In this embodiment any of the known methods (visual, belt with strain gauge etc.) can be used to assess the response of the patients breathing to the PNS stimulus and see if the breathing guidance was successful. In this embodiment the PNS can be again be applied multiple times to the same or different parts of the body in a fairly periodic fashion at the desired breathing rate of the patient. Also the intensity of the PNS (the current amplitude in the coils) can be altered to suggest to the patient that for example they are no longer following the guidance properly.

Embodiment 6: Nerve and muscle stimulation for calming of nervous patient In the sixth embodiment the tactile sensation induced by PNS is used to act as a stimulus to calm an anxious patient in the bore of the MRI scanner without having to physically touch the patient. In this embodiment any of the known methods (GSR, heart rate variation, visual etc.) can be used to assess the response of the patient's anxiety level to the PNS stimulus and see if the calming stimulus was successful. In this embodiment the PNS can be again applied multiple times to the same or different parts of the body. Also the intensity of the PNS (the current amplitude in the coils) can be altered to suggest to the patient that for example a caregiver is stroking their arm.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A stimulation apparatus (10), comprising:
- a magnetic stimulation unit (20); and
- a processing unit (30);
wherein, the processing unit is configured to control the magnetic stimulation unit to provide intentional nerve and/or muscle stimuli to a peripheral body part of a patient.

2. Stimulation apparatus according to claim 1, wherein the apparatus comprises at least one magnetic stimulation coil (50), and wherein the processing unit is configured to control the at least one magnetic stimulation coil to provide the intentional nerve and/or muscle stimuli to the patient in a predefined spatial and/or temporal manner.

3. Stimulation apparatus according to claim 2, wherein the processing unit is configured to select at least one part of one magnetic stimulation coil of the at least one magnetic stimulation coil to provide the intentional nerve and/or muscle stimuli to the patient in the predefined spatial manner.

4. Stimulation apparatus according to any of claims 2-3, wherein the at least one magnetic stimulation coil comprises a plurality of magnetic stimulation coils, and wherein the processing unit is configured to select at least a part of one or more magnetic stimulation coils of the plurality of magnetic stimulation coils to provide the intentional nerve and/or muscle stimuli to the patient in the predefined spatial manner.

5. Stimulation apparatus according to any of claims 2-4, wherein the processing unit is configured to control the magnetic stimulation unit to provide intentional nerve and/or muscle stimuli to a plurality of different locations of the patient.

6. Stimulation apparatus according to any of claims 2-5, wherein the processing unit is configured to control a waveform of a current applied to the at least one magnetic stimulation coil to provide the intentional nerve and/or muscle stimuli to the patient in the predefined temporal manner.

7. Stimulation apparatus according to any of claims 2-6, wherein the apparatus comprises a plurality of magnetic stimulation coil drive amplifiers (60), and wherein the at least one magnetic stimulation coil comprises a plurality of magnetic stimulation coils, wherein each magnetic stimulation coil is configured to be driven by at least one amplifier, wherein each amplifier is configured to drive only one magnetic stimulation coil, and wherein the processing unit is configured control the plurality of amplifiers to provide the intentional nerve and/or muscle stimuli to the patient in the predefined spatial and/or temporal manner.

8. Stimulation apparatus according to claim 7, wherein a first magnetic stimulation coil is configured to be driven by a first amplifier and a second magnetic stimulation coil is configured to be driven by a second amplifier; and optionally a third magnetic stimulation coil is configured to be driven by a third amplifier.

9. Stimulation apparatus according to any of claims 1-8, wherein the processing unit is configured to control the magnetic stimulation unit to provide intentional nerve and/or muscle stimuli to the patient to provide information to the patient.

10. Stimulation apparatus according to any of claims 1-9, wherein the apparatus is configured to acquire at least one patient response to the nerve and/or muscle stimuli; and wherein the processing unit is configured to determine a sedation state of the sedated patient comprising utilization of the at least one patient response to the nerve and/or muscle stimuli.

11. An image acquisition system (100), comprising:
- an image acquisition unit (110); and
- a stimulation apparatus (10) according to any of claims 1-10;
wherein, the image acquisition unit is configured to acquire image data of a patient; and
wherein, the stimulation apparatus is configured to provide intentional nerve and/or muscle stimuli to a peripheral body part of the patient.

12. A magnetic resonance imaging system (200) comprising a stimulation apparatus (10) according to any of claims 1-10, wherein the magnetic resonance image acquisition system is configured to acquire image data of the patient, and wherein the processing unit of the stimulation apparatus is configured to interleave a waveform used for the intentional nerve and/or muscle stimuli of the stimulation apparatus with a waveform used for Magnetic Resonance imaging of the magnetic resonance imaging system.

13. A method (300) of stimulating a patient, comprising:
controlling (310) by a processing unit a magnetic stimulation unit to provide intentional nerve and/or muscle stimuli to a peripheral body part of a patient.

14. A method (500) of image acquisition with a magnetic resonance imaging system, the method comprising:
providing (510) intentional nerve and/or muscle stimuli to a patient by a magnetic stimulation unit according to the method of claim 13;
acquiring (520) image data of the patient with the magnetic resonance imaging system; and
interleaving (530) by the processing unit a waveform used for the intentional nerve and/or muscle stimuli with a waveform used for Magnetic Resonance imaging.

15. A computer program element for controlling an apparatus according to any of claims 1-10 and/or system according to claim 11, which when executed by a processor is configured to carry out the method of claim 13, and/or for controlling a system according to claim 12, which when executed by a processor is configured to carry out the method of claim 14.
